# EUROPEAN PATENT SPECIFICATION

(11) **EP 3 610 259 B1**
(45) Date of publication and mention of the grant of the patent: **12.02.2025**
(21) Application number: 18722811.9
(22) Date of filing: 13.04.2018
(51) Int. Cl.: G01N 33/50, A61B 5/00, G01N 33/543, B82Y 5/00

(54) **HIGH-BRIGHTNESS FLUOROPHORES**
FLUOROPHOREN MIT HOHER HELLIGKEIT
FLUOROPHORES À HAUTE LUMINOSITÉ

(30) Priority: 13.04.2017 US 201762485379 P
(43) Date of publication of application: 19.02.2020
(73) Proprietor: Michigan Technological University, Houghton, Michigan 49931 (US)
(72) Inventor: YAP, Yoke Khin, Houghton, Michigan 49931 (US); ZHANG, Dongyan, Houghton, Michigan 49931 (US); YAPICI, Nazmiye Bihter, South Lyon, Michigan 48178 (US)
(74) Representative: Modiano, Gabriella Diana
(86) International application number: PCT/US2018/027612
(87) International publication number: WO 2018/191690

(56) References cited:
- US-A1- 2015 056 142
- CIOFANI GIANNI ET AL: "Transferrin-conjugated boron nitride nanotubes: Protein grafting, characterization, and interaction with human endothelial cells", INTERNATIONAL JOURNAL OF PHARMACEUTICS, vol. 436, no. 1-2, 23 June 2012 (2012-06-23), NL, pages 444 - 453, XP055851624, ISSN: 0378-5173, DOI: 10.1016/j.ijpharm.2012.06.037
- CIOFANI GIANNI ET AL: "Folate Functionalized Boron Nitride Nanotubes and their Selective Uptake by Glioblastoma Multiforme Cells: Implications for their Use as Boron Carriers in Clinical Boron Neutron Capture Therapy", NANOSCALE RESEARCH LETTERS, vol. 4, no. 2, 25 November 2008 (2008-11-25), pages 113 - 121, XP055851623, DOI: 10.1007/s11671-008-9210-9
- XING CHEN ET AL: "Boron Nitride Nanotubes Are Noncytotoxic and Can Be Functionalized for Interaction with Proteins and Cells", JOURNAL OF THE AMERICAN CHEMICAL SOCIETY, vol. 131, no. 3, 28 January 2009 (2009-01-28), pages 890 - 891, XP055012679, ISSN: 0002-7863, DOI: 10.1021/ja807334b
- KEVIN WELSHER ET AL: "A route to brightly fluorescent carbon nanotubes for near-infrared imaging in mice", NATURE NANOTECHNOLOGY, vol. 4, no. 11, 11 October 2009 (2009-10-11), GB, pages 773 - 780, XP055483894, ISSN: 1748-3387, DOI: 10.1038/nnano.2009.294
- MIAO WENJUN ET AL: "Structure-dependent photothermal anticancer effects of carbon-based photoresponsive nanomaterials", BIOMATERIALS, ELSEVIER SCIENCE PUBLISHERS BV., BARKING, GB, vol. 35, no. 13, 7 February 2014 (2014-02-07), pages 4058 - 4065, XP028668190, ISSN: 0142-9612, DOI: 10.1016/J.BIOMATERIALS.2014.01.043
- KIM MI-GYEONG ET AL: "Polyaptamer DNA nanothread-anchored, reduced graphene oxide nanosheets for targeted delivery", BIOMATERIALS, ELSEVIER SCIENCE PUBLISHERS BV., BARKING, GB, vol. 48, 11 February 2015 (2015-02-11), pages 129 - 136, XP029141976, ISSN: 0142-9612, DOI: 10.1016/J.BIOMATERIALS.2015.01.009
- SAAD M ET AL: "Receptor targeted polymers, dendrimers, liposomes: Which nanocarrier is the most efficient for tumor-specific treatment and imaging?", JOURNAL OF CONTROLLED RELEASE, ELSEVIER, AMSTERDAM, NL, vol. 130, no. 2, 10 September 2008 (2008-09-10), pages 107 - 114, XP026585864, ISSN: 0168-3659, [retrieved on 20080625], DOI: 10.1016/J.JCONREL.2008.05.024

## Description

### BACKGROUND

Fluorophores are compounds with fluorescent properties that have biomedical applications. For example, fluorophores can be used as tracers or dyes for staining certain molecules or structures. More particularly, fluorophores can be used to stain tissues, cells, or materials in a variety of analytical methods, such as fluorescent imaging and spectroscopy.

Fluorophores may be attached to other molecules for delivery to certain tissues, cells or materials. When attached to these other delivery molecules, fluorophores can exhibit quenching, which is a reduction in the brightness of the fluorescence of the fluorophore.

### SUMMARY

An example fluorophore according to the present application includes a carrier which is a boron nitride nanotube, at least one fluorescent entity, and an amphiphilic linker linking each of the at last one fluorescent entities to the carrier. The linker has a molecular weight greater than 1000 Da and/or a stretched linker length greater than 5 nanometers. The linker comprises at least one hydrophilic portion and a hydrophobic portion, wherein the hydrophobic portion is non-covalently bonded to the carrier and the hydrophilic portion is covalently bonded to the fluorescent entity.

Any references in the description to carbon nanotubes (CNT) are for reference purposes only and do not form part of the invention.

### BRIEF DESCRIPTION OF THE DRAWINGS

Figure 1A schematically shows dye-linker structures.
Figure 1B schematically shows fluorophores.
Figure 2A schematically shows an example dye-linker structure of a fluorophore.
Figure 2B schematically shows an example fluorophore with the dye-linker structure of Figure 2A.
Figure 3 shows Quantum Yield plotted against various molecular weight dye-linker structures for the example red dye-linker structure of Figure 2A.
Figure 4A shows Quantum Yield plotted against various molecular weight dye-linker structures for the example red fluorophore of Figure 2B.
Figure 4B shows labelling efficiency plotted against various molecular weight dye-linker structures for the example red fluorophore of Figure 2B.
Figure 5A shows fluorescence intensity plotted against dye concentration for the example red dye-linker structure of Figure 2A.
Figure 5B shows fluorescence intensity plotted against dye concentration for the example red fluorophore of Figure 2B.
Figure 6A shows extinction coefficient plotted against various molecular weight dye-linker structures for the example fluorophore of Figure 2B.
Figure 6B shows brightness plotted against various molecular weight dye-linker structures for the example red fluorophore of Figure 2B.
Figure 7A shows extinction coefficient plotted against various molecular weight dye-linker structures for the example red dye-linker structure of Figure 2A.
Figure 7B shows the brightness plotted against various molecular weight dye-linker structures for the example red dye-linker structure of Figure 2A.
Figure 8A shows another example dye-linker structure.
Figure 8B shows another example fluorophore with the dye-linker structure of Figure 8A.
Figure 9 shows Quantum Yield plotted against various molecular weight dye-linker structures for the example green dye-linker structure of Figure 8A.
Figure 10 shows Quantum Yield plotted against various molecular weight dye-linker structures for the example green fluorophore of Figure 8B.
Figure 11 shows labelling efficiency plotted against various molecular weight dye-linker structures for the example green fluorophore of Figure 8B.
Figure 12A shows another example dye-linker structure.
Figure 12B shows another example fluorophore with the dye-linker structure of Figure 12A.
Figure 13 shows Quantum Yield plotted against various molecular weight dye-linker structures for the example far-red dye-linker structure of Figure 12A.
Figure 14 shows Quantum Yield plotted against various molecular weight dye-linker structures for the example far-red fluorophore of Figure 12B.
Figure 15 shows labelling efficiency plotted against various molecular weight dye-linker structures for the example far-red fluorophore of Figure 12B.
Figure 16 shows extinction coefficient plotted against dye concentration for the example far-red fluorophore of Figure 12B.
Figure 17A shows brightness plotted against various molecular weight linkers for the example green fluorophore of Figure 8B.
Figure 17B shows extinction coefficient plotted against various molecular weight linkers for the example green fluorophore of Figure 8B.
Figure 18A shows brightness plotted against various molecular weight linkers for the example far-red fluorophore of Figure 12B.
Figure 18B shows extinction coefficient plotted against various molecular weight linkers for the far-red example fluorophore of Figure 12B.

### DETAILED DESCRIPTION

Very generally, high-brightness fluorophores contain a carrier element, a fluorescent element, and a linker linking the carrier element to the fluorescent element. For biomedical applications, each of the carrier element, the linker, and the fluorescent element must be biocompatible (though the requirements for biocompatibility will vary with the particular application).

One example carrier element is a nanomaterial, such as carbon nanotubes (CNT) and boron nitride nanotubes (BNNTs), both of which are recognized as biologically compatible nanomaterials for biomedical applications such as cellular drug delivery and spectroscopy applications. However, it has been shown that fluorescent elements linked to nanotubes exhibit quenching, or a reduction in the brightness of the fluorescence.

It has been discovered that certain fluorophores having nanomaterial carriers not only do not exhibit the quenching effect, but also exhibit brightness several orders of magnitude higher than other known fluorophores, as will be discussed herein.

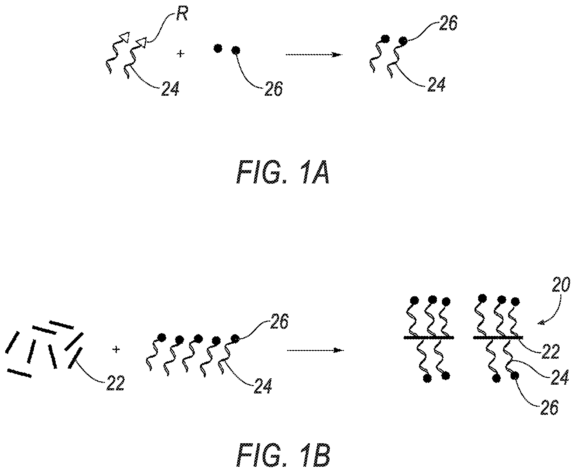

Referring now to Figures 1A-B, fluorophores 20 are schematically shown. Fluorophores 20 generally comprise an inorganic nano-scale carrier 22, a linker 24, and a fluorescent entity 26.

The carrier 22 is a BNNT carrier. In a particular example, the carrier 22 is a multi-walled BNNT carrier, where each BNNT has multiple co-axial shells of hexagonal boron nitride (h-BN for BNNTs), with a typical external diameter of more than about 5 nm but less than about 80 nm. The length of these BNNTs may be between about 50-1000 nm. The carrier 22 can be fabricated by any known method.

The linker 24 is an amphiphilic polymeric linker, linking each of the at least one fluorescent entities to the carrier, the linker having a molecular weight greater than 1000 Da and/or a stretched linker length greater than 5 nanometers. The linker 24 includes a hydrophobic region 28 and a hydrophilic region 30. The hydrophobic region 26 non-covalently bonds to the nanotube carrier 22, while the hydrophilic region is covalently bonded to the fluorescent entity 26. One example linker 24 is DSPE-PEGₙ (1,2-distearoyl-sn-glycero-3-phosphoethanolamine-N-[(polyethylene glycol)ₙ]), where n is a number of polyethylene glycol (PEG) molecules in a PEG chain. Other linkers 24 can similarly include a PEG chain (or a different chain) which varies in length.

In addition to the DSPE-PEG linkers 24 discussed above, many other potential linkers are known in the art. For example, a linker 24 may comprise one or more groups selected from -CH₂-, -CH=, -C=, -NH-, -N=, O-, -NH₂-, -N₃-, -S-, -C(O)-, -C(O)₂-, - C(S)-, -S(O)-, -S(0)₂-, or any combination thereof. It will be appreciated that a linker comprising more than one of the above groups will be selected such that the linker 24 is stable; for example, a linker 24 may not include two adjacent -O- groups, which would generate an unstable peroxide linkage. The linker 24 may be a straight chain, a branched chain, or may include one or more ring systems. Non-limiting exemplary linkers include a hydrophobic area which can be fatty acids, phospholipids, sphingolipids, phosphosphingolipids [such as DSPE, 1-O-hexadecanyl-2-O-(9Z-octadecenyl)-sn-glycero-3-phospho-(1'-rac-glycerol) (ammonium salt), N-octanoyl-sphingosine-1-{succinyl[methoxy(polyethylene glycol)5000, D-erythro-sphingosyl phosphoethanolamine, 1,2-diphytanoyl-sn-glycero-3-phospho-L-serine, 3-sn-phosphatidyl-L-serine (PS), glycosylphosphatidylinositol,1,2-dioleoyl-sn-glycero-3-phosphoethanoamine but not limited). The hydrophobic unit can be used to conjugate with water soluble polymeric chains such as PEG (or PEO polyethyleneoxide), PMO (poly methyl oxazoline), PEI (polyethyleneimine), polyvinyl alcohol, polyvinylpyrolidone, polyacrylamide, polypeptide, carbohydrate anchors. The water soluble polymeric chains are attached to the linkers at one end, and attached to the fluorescent entity at a second end. These hydrophobic and hydrophilic units must have reactive groups as mentioned above and such that the groups conjugate together into amphiphilic linkers.

The fluorescent entity 26 is any know fluorescent dye, including but not limited to coumarins, benzoxadiazoles, acridones, acridines, bisbenzimides, indole, benzoisoquinoline, naphthalene, anthracene, xanthene, pyrene, porphyrin, fluorescein, rhodamine, boron-dipyrromethene (BODIPY) and cyanine derivatives. Many such fluorescent dyes are commercially available. The fluorescent entity 26 is bonded to the linker 24 by any appropriate method.

Generally, the brightness of the fluorophore 20 is directly related to the number of fluorescent entities 26 on the fluorophore 20. That is, a fluorophore 20 with less fluorescent entities 26 will exhibit a lower brightness than a fluorophore 20 with more fluorescent entities 26. However, it has also been discovered that linker 24 length also affects the brightness of the fluorophore 20. In the particular example DSPE-PEGₙ linker 24 discussed above, varying the number of PEG molecules in the PEG chain (n) varies the length of the linker 24, and thus the brightness of the fluorophore 20. It will be appreciated that varying linker lengths of the other types of linkers discussed above can also be achieved. More particularly, it has been discovered that fluorophores 20 having linker 24 molecular weight of greater than about 1000 Da (which corresponds to a stretched linker length of about 5-10 nm for a linker 24 with a PEG chain) exhibit a nonlinear quenching effect, which is unexpected. Accordingly, the fluorophores 20 described herein exhibit brightness several orders of magnitude higher than prior art fluorophores. Furthermore, it has been discovered that fluorophores 20 with different fluorescent entities 26 may have a different relationship between their fluorescent properties and linker 24 length.

In one example, the linker 24 can include a functional group R. The functional group R is a reactive group that facilitates covalent bonding of the linker 24 to the fluorescent entity 26 by know chemistry. An example functional group R is an amine group. Other example functional groups are carboxylic acid, isothiocyanate, maleimide, an alkynyl group, an azide group, a thiol group, monosulfone, or an ester group such as a succinimidyl, sulfodichlorophenol, pentafluorophenyl or tetrafluorophenyl. The functionalized linker 24 (that is, a linker 24 with a functional group R) may be commercially available, or may be synthesized according to methods described herein or other methods known to those skilled in the art.

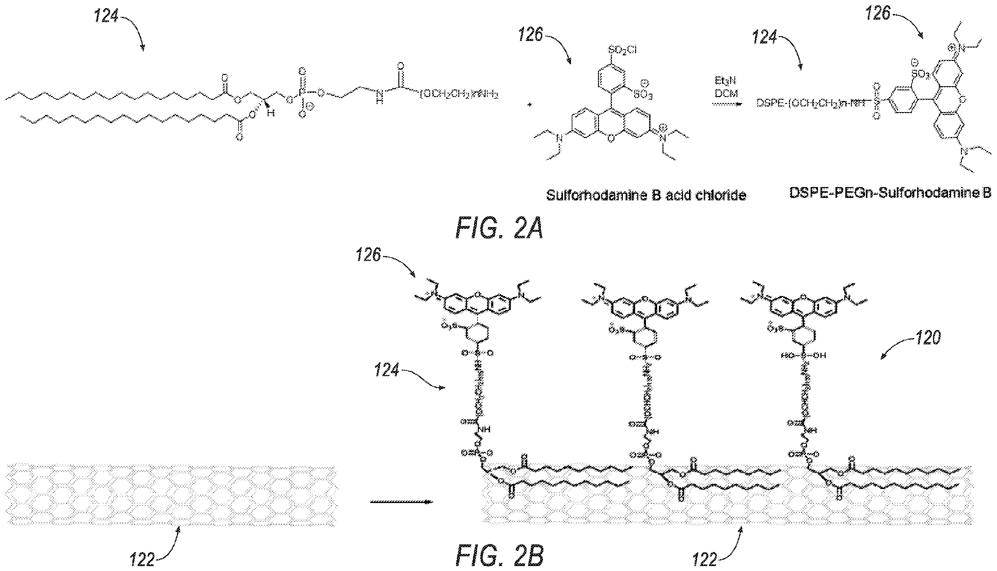

Figures 2A-B show an example red fluorophore 120. The example red fluorophore 120 includes a BNNT carrier 122, an amide-functionalized DSPE-PEGn-NH₂ linker 124 (that is, a linker as discussed above with an amide functional group, NH₂), and a sulforhodamine B (RhB, red) fluorescent entity 126.

The RhB fluorescent dye entity 126 is covalently bonded to the DSPE-PEGn-NH₂ linker 124 by any method to form a dye-linker structure 124, 126 as shown in Figure 2A. For example, the RhB fluorescent entity 126 is combined with DSPE-PEGn-NH₂ linker 124 in an ice bath under nitrogen in anhydrous dichloromethane (DCM), and then purified by flash chromatography or another purification method.

The use of DSPE-PEGn-NH₂ linker 124 with various molecular weight (MW) PEG chains (that is, with various n values) causes dye-linker structure 124, 126 to emit at different fluorescence intensity and fluorescence quantum yield (QY). Since the dye entity 126 is at the end of the PEG chain of the linker 124 opposite the linker 124 connection to the BNNT carrier 122, higher MW of the PEG chain (and higher n values) means the linker 124 is longer, and thus that the dye entity 126 would be further from the BNNT carrier 122. Though the below description is made with respect to the particular example red fluorophore 120, it should be understood that it is also applicable to fluorophores 20 including other linkers, carriers, or fluorescent entities, as discussed above.

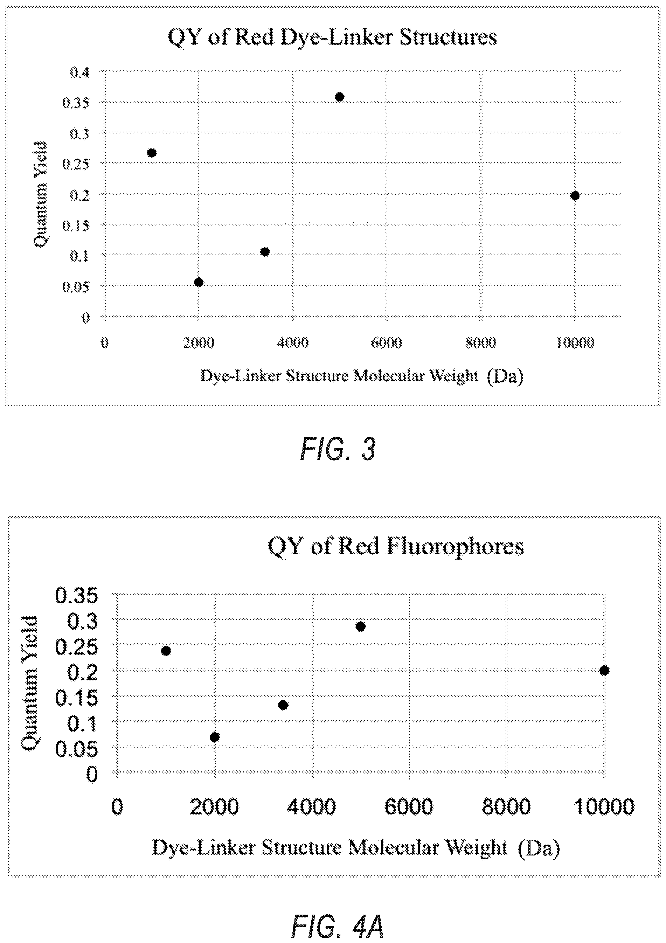

Figure 3 shows the QY for the dye-linker structure 124, 126 with various PEG MW (1000, 2000, 3400, 5000, and 10,000 Da, corresponding to n= 22, 45, 77, 114, 227 PEG molecules, respectively). The length of a fully stretched PEGn chain can be estimated because the known length of one PEG entity is 0.44nm. For example, a fully stretched 5000 MW PEG chain is calculated as 5,000/44 × 0.44 nm = 50 nm. The fully stretched linker lengths for PEG chains with MW of 1000, 2000, 3400, 5000, 10,000 are estimated to be 5-10nm, 11-20nm, 18-34nm, 27-50nm, and 54-100nm, respectively. In reality, the PEG chains may be coiled about one another or themselves, and may not retain their fully stretched state.

The relative fluorescence QY for each dye-linker sample was calculated by QY (sample) = QY (Reference) x [Slope(sample)/Slope(reference)] x [r(sample)/r(reference)], where r is the refractive index. As indicated by the equation, the relative QY was independent of the dye concentration of the samples as it was calculated by fluorescence/absorbance slope ratio of the samples and reference which were both linearly scaled to concentration as shown in Figure 3.

As shown in Figure 3, it is surprising to see that the QY changes for various linker 124 MW (e.g., various linker 124 length). In particular, for the example fluorophore 120, the QY increases in a nonlinear manner with the linker length for MW of 2000 to 5000 but is saturated of decreasing at MW by 10,000 (that is at MW 10000, a longer linker does not cause a higher QY). It is also unexpected to see that the QY at MW of 1000 was higher than the QY at MW of 2000 and 3400. The maximum QY detected in the case of MW=5000 was also close to the standard QY free RhB (~0.31 in distilled water), which indicates that fluorescence quenching is absent.

For example fluorophore 120, the dye-linker structure 124, 126 (DSPE-PEGn-NH₂-RhB) is non-covalently labeled on the BNNT carrier 122 as shown in Figure 2B by any method. The BNNT carrier 122 is fabricated and cut by any known method to a desired length. For example, the BNNT carrier 122 is between about 50 and 1000 nm, more particularly, between about 300 and 400 nm. The BNNT carrier 122 is exposed to the dye-linker structure 124, 126 so that the dye-linker structure 124, 126 non-covalently bonds to the BNNT carrier 122 by any method. Optionally, the BNNT carrier 122/dye-linker structure 124-126 solution can be distilled or filtered to remove excess unbonded dye-linker structures 124, 126.

As shown in Figure 2B, the alkyl chain (-C(O)(CH₂)₃₄) of the linker 124 is non-covalently adsorbed on the surface of BNNT carrier 122 while the PEGn-NH₂-RhB of the dye-linker structure 124, 126 extends away from the BNNT carrier 122. For other examples, the hydrophobic end of the linker 24 non-covalently bonds to the carrier 22 while the free hydrophilic end is covalently bonded to a fluorescent entity 26, as discussed above.

It is noted that the surface area of one DSPE-PEGn-NH₂ linker 124 molecule adsorb on a BNNT is 1.44 nm². This means there can be as many as 1.36×10⁶ DSPE-PEGn-NH₂-RhB dye-linker structures 124, 126 on a single BNNT carrier 122 that is 500 nm long and 50 nm in diameter if all the dye-linker structures 124, 126 are lined up in a straight line. Accordingly, the fluorophore 120 is estimated to have 6 orders of magnitude more fluorescent entities than prior art fluorophores that consist of only 1-6 florescent entities. More generally, it is estimated that the fluorophores 20 described herein include at least 100, and more particularly at least 1000 dye-linker structures 24, 26.

Figure 4A shows the QY of the fluorophores 120 with various dye-linker structures 124, 126 as discussed above. As shown, the trend of the QY is quite similar to that of QY for the dye-linker structures 124, 126 alone, as illustrated in Figure 3.

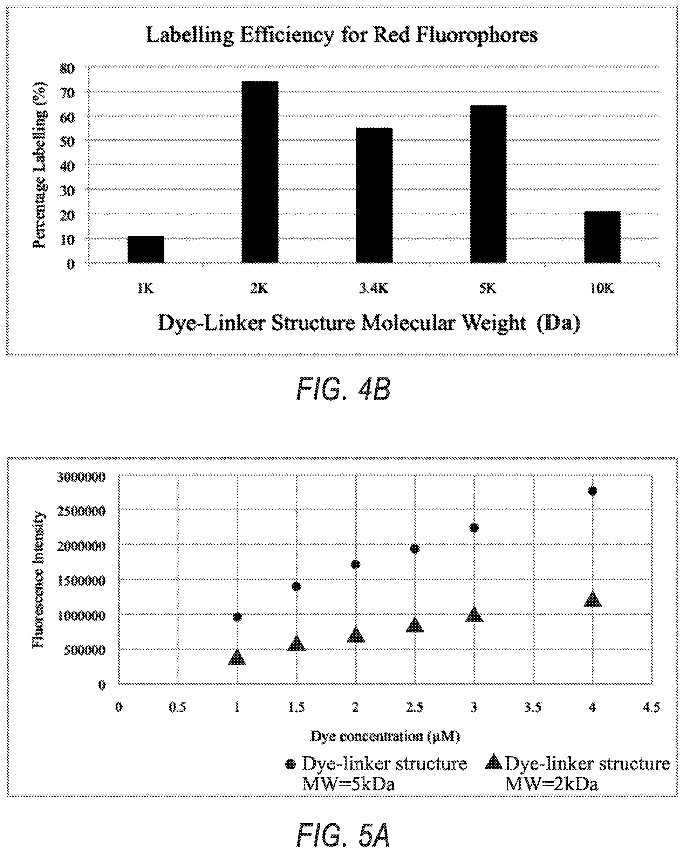

Figure 4B shows the labeling efficiency of the various dye-linker structures 124, 126 discussed above on BNNT carriers 122. The labelling efficiency was calculated by determining the concentrations of dye-linker structures 124, 126 after being labeled on carrier BNNTs 122. This actual concentration of dye-linker structures 124, 126 was then compared to initial dye concentration being used for each labeling process to determine the labeling efficiency.

It is surprising to see that for the example fluorophore 120, labeling efficiencies for small (MW=1000) and large (MW=10000) linkers are significantly low (<20%). The labeling efficiency for linkers with intermediate MW (2000, 3400, 5000) are quite similar in labeling efficiency (55-75%).

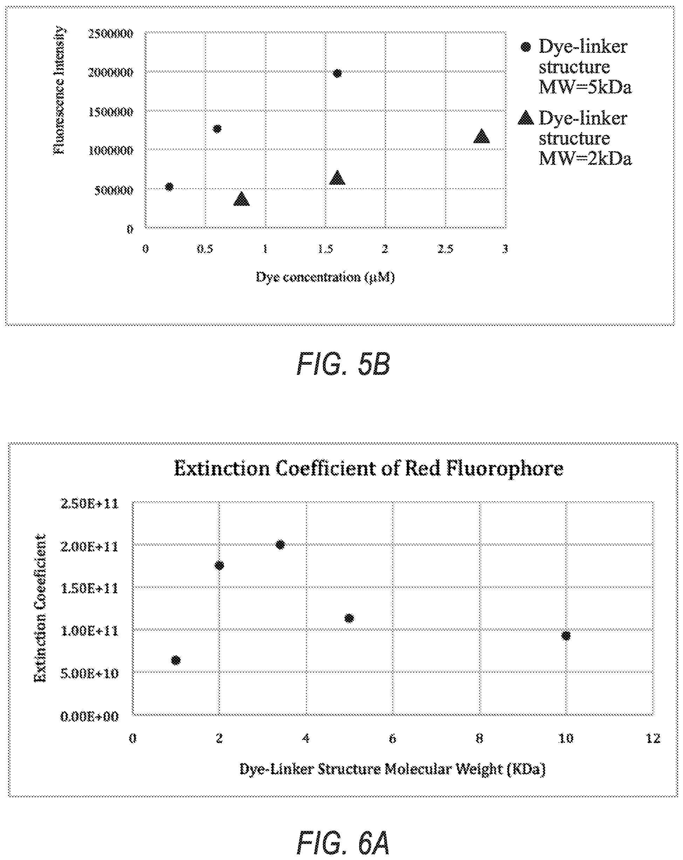

The fluorescence brightness of the fluorophore 120 is also related to the concentration of dye-linker structures 124, 126 that the BNNT carriers 122 are exposed to. This in turn affects the labelling efficiency, discussed above, and ultimately the number of fluorescent dye entities 126 on each BNNT carrier 122. Figure 5A shows a plot of fluorescence intensity versus concentration of dye-linker structures 124, 126 themselves, while Figure 5B shows a plot of fluorescence intensity versus concentration of dye-linker structures 124, 126 for fluorophores 120.

As shown in Figure 5B, it is unexpected to see that large quantity of dye-linker structures 124, 126, e.g., at high concentrations, can be labeled on BNNT carriers 120 without noticeable decrease in fluorescence intensity. This means, stable and non-covalent bonding between BNNT carrier 120 and the dye-linker structures 124, 126 can prevent aggregation and collisional quenching and therefore lead to controllable and enhanced fluorescence brightness by using more concentrated dye-linkers for the labeling.

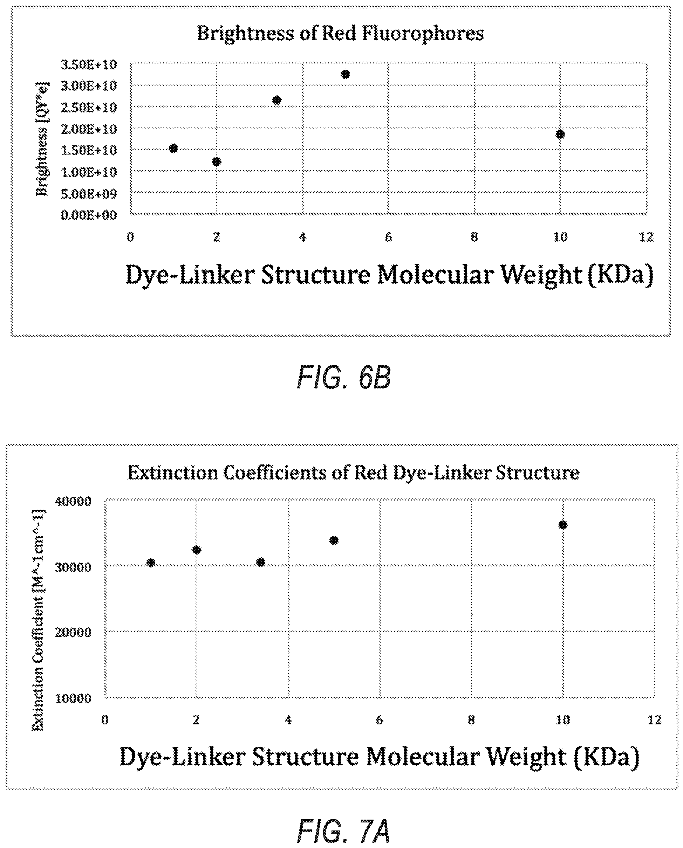

Brightness of fluorophores is defined as product of quantum yield (QY) and extinction coefficient (ε). Since a single BNNT carrier 120 could be loaded as many as 1.5×10⁶ fluorescent entities, as discussed above, the brightness of each of the example fluorophores 120 is several orders of magnitude brighter than prior art fluorophores which have only a few fluorescent dye entities on each fluorophore (e.g., 1-6, as discussed above). In fact, the extinction coefficient for the example fluorophores 120 with various molecular weight linkers 124 are in the range of 1×10⁷ to 1×10¹¹ M⁻¹cm⁻¹ (as shown in Figure 6A), which is much higher than the extinction coefficient of brightest commercial dye (phycoerythrin (PE)) with an extinction coefficient of about 1×10⁶ M⁻¹cm⁻¹. Figure 6B shows the brightness of the fluorophores 120. Figures 7A-B show the extinction coefficient and brightness of the dye-linker structures 124, 126.

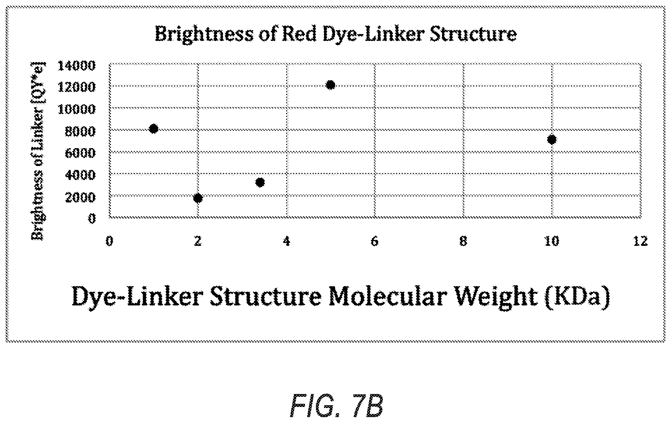

As shown in Figure 6B, the brightness of the example fluorophores 120 are much higher ~10¹⁰ than those of the dye-linker structures 124, 126 shown in Figure 7B. This is due to the high extinction coefficients of the fluorophores 120 for all linker 124 lengths, as compared to those of the free dye-linker structures 124, 126. The extinction coefficient is dependent on the labeling efficiency of the dye-linker structures 124, 126 onto the BNNT carriers 122 (Figure 4B). Therefore, the brightness are highest for the linkers with MW = 3400 and 5000 Da. In any case, the extinction coefficients for the example fluorophores 120 for all linker 124 lengths are several orders of magnitudes higher than those of existing commercial fluorophores.

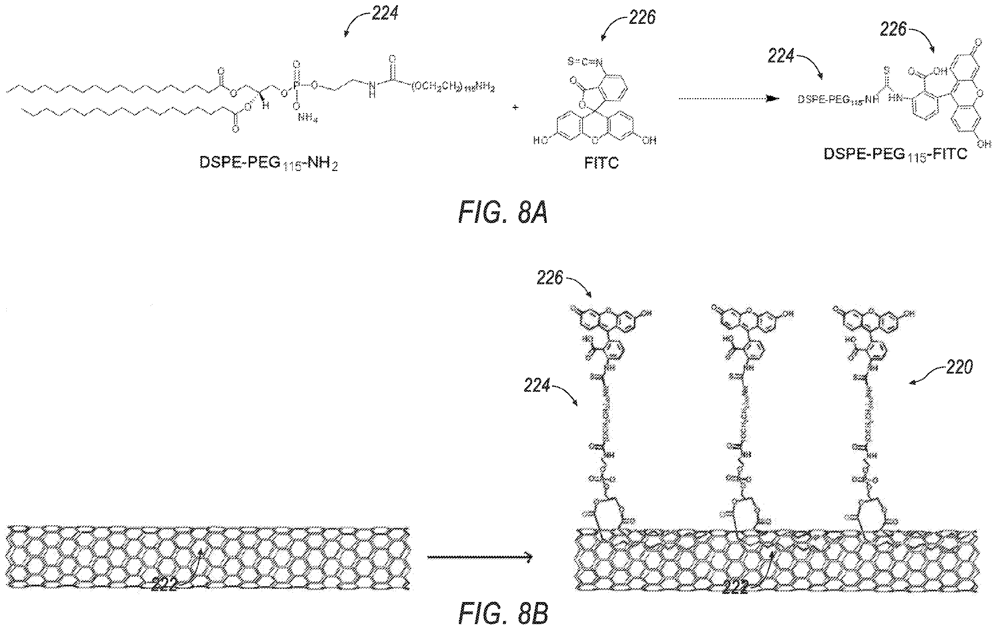

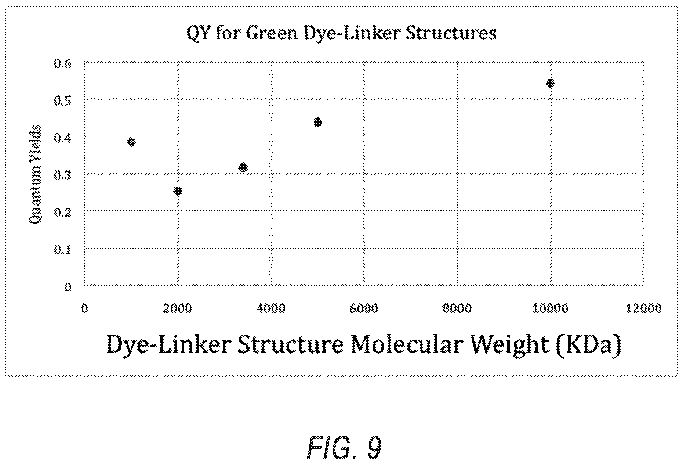

Another example green fluorophore 220, shown in Figures 8A-B includes a nanotube carrier 222 and a DSPE-PEG-NH₂ linker 224, as in the previous example, but includes a fluorescein isothiocyanate (FITC, green) fluorescent entity 226 instead of RhB as in the previous example. Figure 9 shows QY for dye-linker structures 224, 226 for the same molecular weight linkers 224 as in the previous example. As shown in Figure 9, the dye-linker structures 224, 226 exhibit a non-linear trend with linker 224 molecular weight.

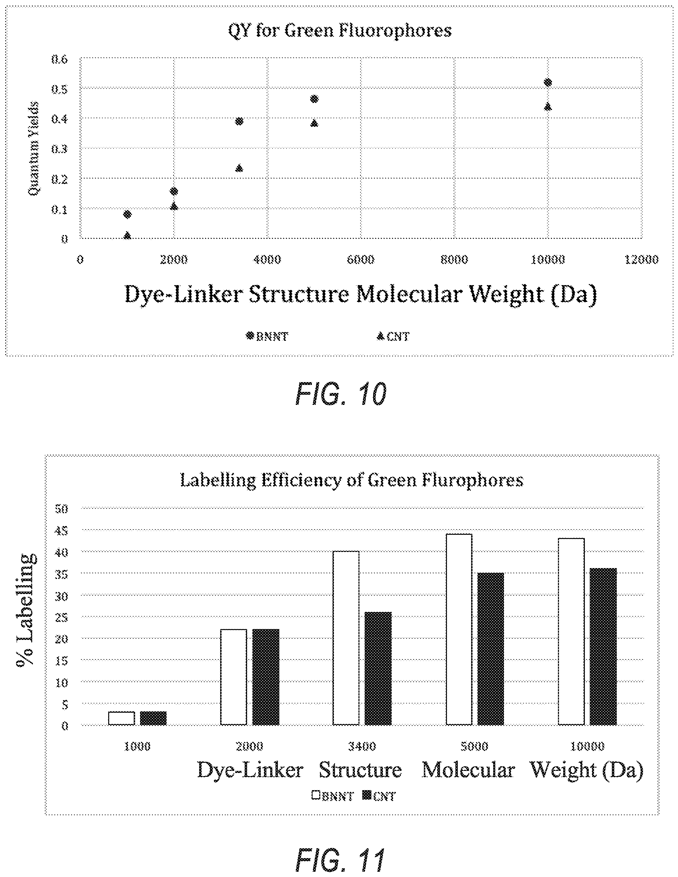

Figure 10 shows QY of dye-linker structures 224, 226 labelled onto two types of nanotube carriers 220, CNTs (reference) and BNNTs. As shown, there is a nominal difference in QY between CNT and BNNT carriers. Also, there is generally a linear trend between linker molecular weight and QY for both CNT and BNNT carriers. The fluorophores 220 exhibited lower QY than laser grade fluorescein was used as reference which is known to have QY of 0.86 in phosphate-buffered saline (PBS) solution. Therefore, the fluorophores 220 exhibited quenching. This could be due to the relatively low labelling efficiency of the dye-linker structures 224, 226 onto the nanotube carriers 222 as compared to the first example fluorophores 120, especially for low molecular weight linkers 224 (shown in Figures 11 and 4B, respectively). It should be noted that FITC is a pH sensitive dye and the low labeling efficiency of these short-length dye-linker structures 224, 226 is affected by the molecular structure of dye-linker structures 224, 226.

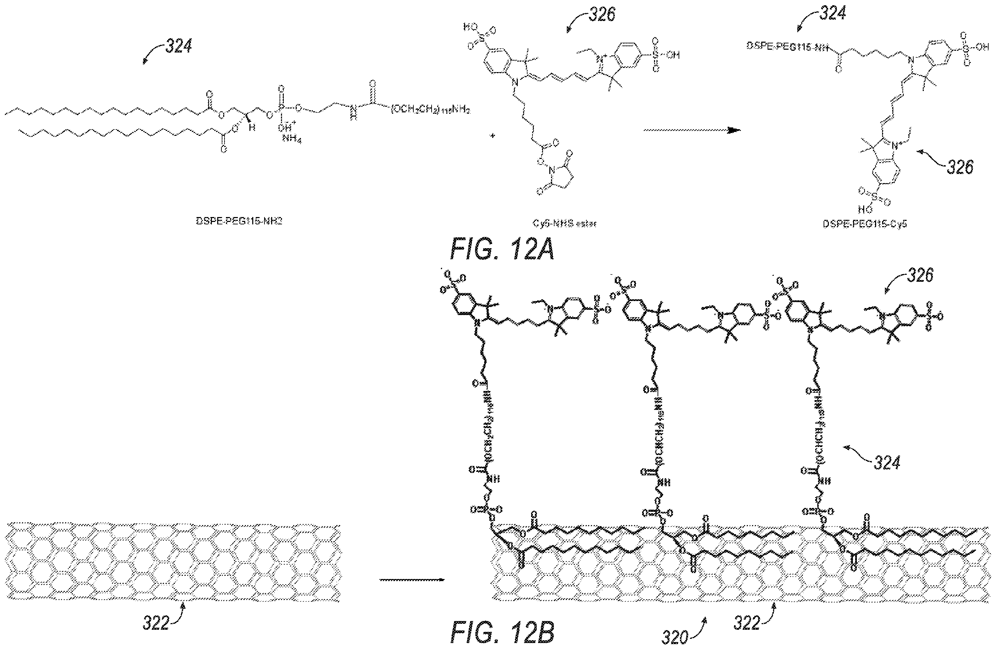

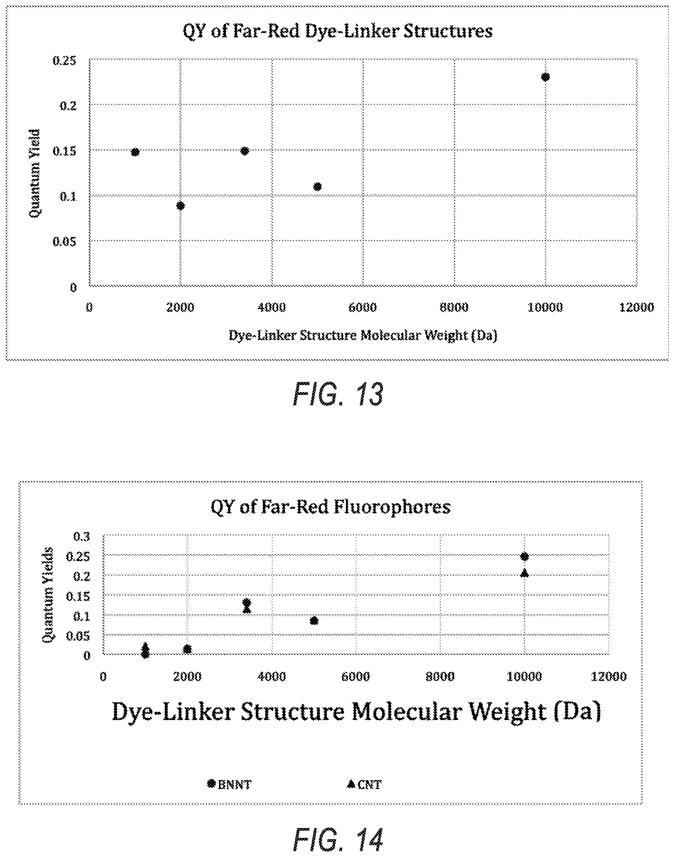

Another example far-red fluorophore 320, shown in Figures 12A-B includes a nanotube carrier 322 and a DSPE-PEG-NH2 linker 324, as in the previous example, but includes a sulfoCy5 (far-red) fluorescent entity 326 instead of RhB or FITC as in the previous examples. Figure 13 shows QY for dye-linker structures 324, 326 for the same molecular weight linkers 224 as in the previous example. As shown in Figure 13, the dye-linker structures 324, 326 exhibit a non-linear trend with linker 324 molecular weight.

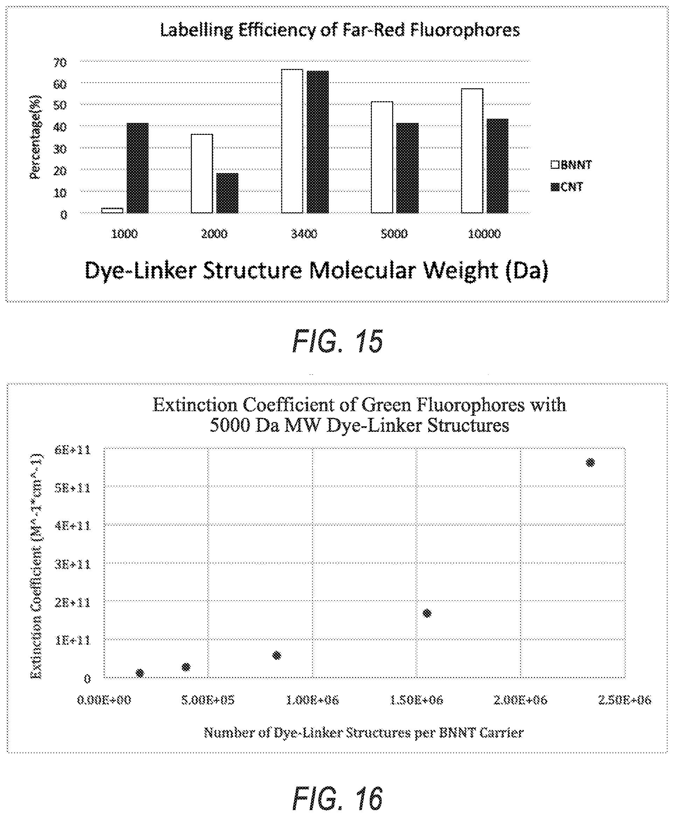

Figure 14 shows QY of dye-linker structures 324, 326 labelled onto two types of nanotube carriers 320, CNTs (reference) and BNNTs. As shown, there is a nominal difference in QY between CNT and BNNT carriers. Also, there is generally a linear trend between linker molecular weight and QY for both CNT and BNNT carriers. The fluorophores 320 exhibited lower QY than a reference dye (3,3'Diethythiadicarbobynine iodine, which is known to have QY of 0.31 in EtOH). Therefore, the fluorophores 320 exhibited quenching, especially for linker 324 MW below 10000. This could be due to the relatively low labelling efficiency of the dye-linker structures 324, 326 onto the nanotube carriers 322 as compared to the first example fluorophores 120, especially for low molecular weight linkers 224 (shown in Figures 15 and 4B, respectively). It should be noted that sulfoCy5 is a small molecule and that Cy5 dyes are known to quench and aggregate at high concentrations.

There were no noticeable spectral peak shift in the absorption spectra of the red fluorophores 120, the green fluorophores 220, or the far-red fluorophores 320. This means, the non-covalent bonding of these dye-linkers structures to the carrier were stable to prevent dye aggregation and collisional quenching and therefore led to the enhanced fluorescence intensity when higher dye-linker concentrations were used in the labeling process. However, this was not the case, when dye-linker length below 3400 for the far-red fluorophores 320. There was significant aggregation.

The green fluorophores 220 and far-red fluorophores 320 exhibited high extinction coefficients within linkers of molecular weight 5000, as estimated from the slope of the absorbance of the FITC entity 226 as a function of dye concentration (which in turn is related to the number of dye-linker structures on each nanotube carrier. Figure 16 shows the extinction coefficient of fluorophores 220 versus number of dye-linker structures 224, 226 per BNNT carrier 222. As shown, the extinction coefficient of these green fluorophores 220 can be as high as 1.65×10¹² to 5.63×10¹³ M⁻¹cm⁻¹. The fluorescence intensity continues to increase linearly with the number of dye-linker structures 224, 226 labeled on BNNT carriers 222 (at the range of 3.96×10⁷ to 3.2×10⁸ dye-linker structures 224, 226 per BNNT carrier 222). This is unexpected as in prior art fluorophores, quenching occurred where more than a few dyes molecules were conjugated in close proximity.

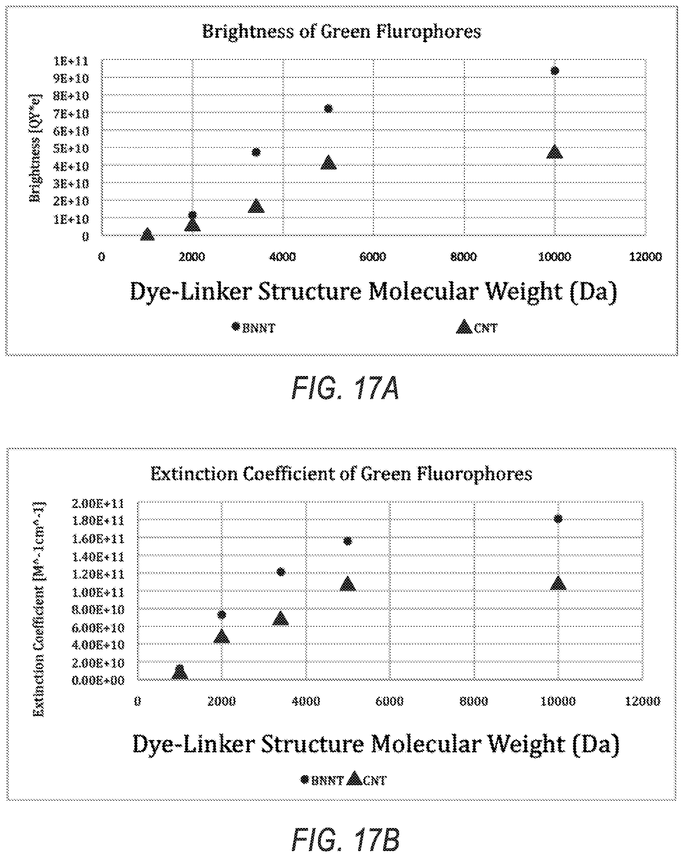

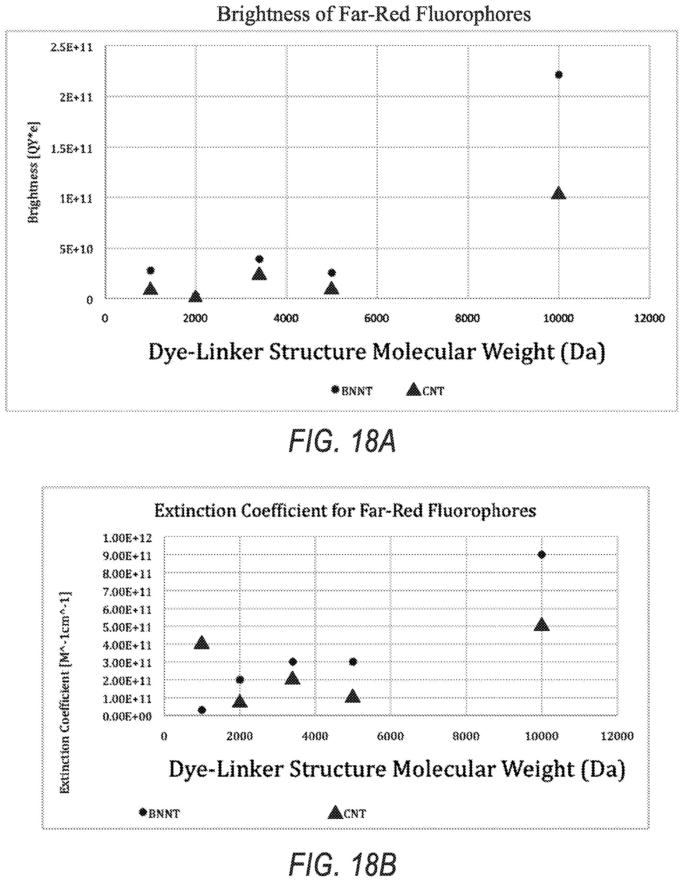

Figures 17A-B and 18A-B show brightness and extinction coefficients of green and far-red fluorophores 220, 320 respectively, for both CNT (reference) and BNNT carriers. As shown, the extinction coefficients for green fluorophores 220 increase with linker MW up to about 2×10¹¹ M⁻¹cm⁻¹ while brightness ranges between about 6×10⁹ and 1×10¹¹. For the far-red fluorophore 320, the extinction coefficients increase with linker MW up to about 1×10¹² M⁻¹cm⁻¹ and brightness ranges between about 4×10¹⁰ and 2×10¹¹.

As discussed above, BNNT and CNT are structurally similar. However, CNTs are electrically conductive, while BNNTs are electrically insulating. When similar sized BNNTs and CNTs are labelled with the same red dye-linker structure, for instance, the example red dye linker structure 124, 126 discussed above, the BNNT fluorophores exhibit a fluorescence intensity about 4.5 times larger than that for similar CNTs. This result suggests that the relative QY of red-fluorophores using BNNTs as the carrier can be 4.5-times higher than the QY of red-fluorophores using CNTs as the carrier.

One explanation for the difference is as follows. It is understood that fluorescent entities in physical contact with an electrically insulating matter will subjected to lower fluorescence quenching as compared to the case when the fluorescence particles are in contact with an electrically conducting matter. However, the fluorescent entity 26 used herein is connected to the carrier 20 through a long polymeric linker 24 e.g., one that is electrically insulating such as the DSPE-PEG linker discussed above. Accordingly, it is expected the linker 24 insulates the fluorescent entity 26 from any effect of the electrical conductivity of a CNT carrier 22. Unexpectedly, the discovered different fluorescent intensities between fluorophores with CNT carriers and BNNT carries implies characteristics of the carrier do affect the fluorescence of a fluorophore. The result also suggests that electrically insulating nanomaterials form higher-brightness fluorophores with high QY than prior art fluorophores. Other electrically insulating nanomaterials that can be used as carrier 22 include BN nanosheets, BN nanoparticles, silica particles, alumina particles, nanowires or nanorods of Si, Ge, ZnO, etc.

Nonetheless, although the relative QY of fluorophores made by using CNTs are 4.5x lower than those made by using BNNTs, the number of dye-linkers per CNT and per BNNT can be identical, as discussed above. Therefore, the extinction coefficients of fluorophores prepared by using CNTs would be the same order of magnitude as those prepared by using BNNTs. Accordingly, high-brightness fluorophores with CNT carriers are still much brighter than other prior art fluorophores.

For the cases of green and far-red labelled fluorophores such as the example fluorophores 220, 320 discussed above, there was no significant QY difference when these dye-linker structures are labelled on BNNT versus and CNT as shown in Figures 10 and 14 . Apparently, the electrically insulating or conducting nature of the nanomaterials (BNNTs and CNTs here) did not affect the QY of FITC and Cy5 fluorescent entities as they did on RhB fluorescent entities. This means, the lengths of linkers (e.g., linkers with MW 1000 or higher) are sufficient to prevent FITC and Cy5 from significant quenching to the nanomaterials of the carrier. All these green and far-red fluorophores are much brighter than commercial dyes due to their high extinction coefficients, as discussed above.

The high-brightness fluorophores described here are photostable even under the irradiation of tightly focused laser under a confocal fluorescence microscopy. For example, red fluorophores 120 in HeLa cells were monitored for five days and did not indicate visible reduction in fluorescence intensity as examined using the same microscopy setting (same focus ratio, same light source power, same gain and same excitation wavelength). This indicates the example fluorophores described herein are more photostable than prior art stains regularly used for cell microscopy imaging. Furthermore, proliferation and signal stability were observed in daughter cells. This result suggests that the structure of the fluorophores described herein is stable and biological compatible such that it can also be used as a photostable stain *in vitro* and *in vivo* for tracking.

Additionally, cells incubated with the red fluorophores 120 described above exhibited a relationship between concentration of dye-linkers and fluorescence intensity. This means fluorescence intensity described above for red, green, and far-red fluorophores has the same trend inside cells and is therefore applicable for *in vitro* and *in vivo* cell tracking application.

Though the example fluorophores described above include only one type of fluorescent entity 26, fluorophores 20 including multiple types of fluorescent entities 26 linked to a single carrier 22 are also contemplated.

Furthermore, any of the fluorophores 20 described herein can also be conjugated with biological molecules such as antibodies in addition to fluorescent entities 26 using the same or different linkers 24. This allows the fluorophores 20 to be simultaneously functionalized with biological molecules for specific biological labeling on cell membranes or other structures inside cells. Antibodies or other biological molecules can be attached to linkers 24 by known methods and chemistries. As discussed above, linkers 24 include R functional groups to facilitate conjugation to other molecules. The R group can be selected according to the biological molecule to be attached to the linker 24. For instance, a monosulfone-thiol reaction can be used to conjugate an antibody to a monosulfone R-group. Malimide R groups can also be used.

In one example, the green fluorophore 220 with 5000 MW linker 224 discussed above was co-labelled anti-human CD4 on BNNT carriers 222. Absorption spectra indicated that the antibody concentration on fluorophore 220 was comparable to that of the commercial FITC fluorophores with CD4. The fluorophore 220 with CD4 had 5X higher fluorescence intensity than the commercial anti-human CD4 FITC compound at 4X lower concentration.

In addition to or instead of antibodies, fluorophores 20 can also be labelled with peptides, oligonucleotides or other macromolecules such as DNA, RNA, antibodies via a linker 24 in the same manner discussed above.

Cross-linkers which contain dual functional groups can also be used to connect the linker 24 to other entities such as fluorescent entities 26, peptides, oligonucleotides, DNA, RNA, antibodies, etc. Example cross-linkers might be SMCC (succinimidyl 4-(N-maleimidomethyl)cyclohexane-1-carboxylate), sulfo-SMCC ((sulfosuccinimidyl 4-(N-maleimidomethyl)cyclohexane-1-carboxylate), AMAS (N-α-maleimidoacet-oxysuccinimide ester), BMPS (N-β-maleimidopropyl-oxysuccinimide ester), GMBS (N-γ-maleimidobutyryl-oxysuccinimide ester), sulfo-GMBS, MBS (m-maleimidobenzoyl-N-hydroxysuccinimide ester), sulfo-MBS, EMCS (N-ε-malemidocaproyl-oxysuccinimide ester), sulfo-EMCS, SMPB (succinimidyl 4-(p-maleimidophenyl)butyrate), sulfo-SMPB, SMPH (Succinimidyl 6-((beta-maleimidopropionamido)hexanoate), LC-SMCC succinimidyl 4-(N-maleimidomethyl)cyclohexane-1-carboxy-(6-amidocaproate), sulfo-KMUS (N-κ-maleimidoundecanoyl-oxysulfosuccinimide ester), SM(PEG)n where n=2,4,6,8,12,24 (PEGylated SMCC cross-linker), SPDP (succinimidyl 3-(2-pyridyldithio)propionate), LC-SPDP, sulfo-LC-SPDP, SMPT (4-succinimidyloxycarbonyl-alpha-methyl-α(2-pyridyldithio)toluene), PEGn-SPDP (where n= 2,4,12, 24), SIA (succinimidyl iodoacetate), SBAP (succinimidyl 3-(bromoacetamido)propionate), SIAP (succinimidyl (4-iodoacetyl)aminobenzoate), sulfo-SLAP, ANB-NOS (N-5-azido-2-nitrobenzoyloxysuccinimide),sulfo-SANPAH (sulfosuccinimidyl 6-(4'-azido-2'-nitrophenylamino)hexanoate), SDA (succinimidyl 4,4'-azipentanoate), sulfo-SDA, LC-SDA, sulfo-LC-SDA, SDAD (succinimidyl 2-((4,4'-azipentanamido)ethyl)-1,3'-dithiopropionate), Sulfo-SDAD, DCC (N,N'-Dicyclohexylcarbodiimide), EMCH (N-ε-maleimidocaproic acid hydrazide), MPBH (4-(4-N-maleimidophenyl)butyric acid hydrazide), KMUH (N-κ-maleimidoundecanoic acid hydrazide), PDPH (3-(2-pyridyldithio)propionyl hydrazide), PMPI (p-maleimidophenyl isocyanate), SPB (succinimidyl-[4-(psoralen-8-yloxy)]-butyrate).

## Claims

1. A fluorophore, comprising:
a carrier which is a boron nitride nanotube;
at least one fluorescent entity; and
an amphiphilic linker linking each of the at last one fluorescent entities to the carrier, the linker having a molecular weight greater than 1000 Da and/or a stretched linker length greater than 5 nanometers, wherein the linker comprises at least one hydrophilic portion and a hydrophobic portion, and wherein the hydrophobic portion is non-covalently bonded to the carrier and the hydrophilic portion is covalently bonded to the fluorescent entity.

2. The fluorophore of claim 1, wherein the hydrophilic portion includes a chain of water soluble polymeric molecules attached to the hydrophobic portion at a first end, and the at least one fluorescent entity is attached to the chain at a second end; more preferably wherein the linker comprises a DSPE (1,2-distearoyl-sn-glycero-3-phosphoethanolamine) group and a chain of polyethylene glycol (PEG) molecules.

3. The fluorophore of claim 1, wherein the at least one fluorescent entity comprises at least one of a coumarin, a benzoxadiazole, an acridone, an acridine, a bisbenzimide, indole, benzoisoquinoline, naphthalene, anthracene, xanthene, pyrene, porphyrin, fluorescein, rhodamine, boron-dipyrromethene (BODIPY), and a cyanine derivative.

4. A fluorophore according to claim 1, comprising:
a boron nitride nanotube;
at least one amphiphilic linker having a hydrophobic portion and a hydrophilic portion, wherein the hydrophobic portion is non-covalently bonded to the nanotube, wherein the at least one amphiphilic linker has a molecular weight greater than 1000 Da; and
at least one fluorescent entity covalently bonded to the hydrophilic portion of the at least one amphiphilic linker.

5. The fluorophore of claim 4, wherein the hydrophilic portion includes a chain of water soluble polymeric molecules.

6. The fluorophore of claim 5, wherein the water soluble polymeric molecules are polyethelene glycol (PEG) molecules.

7. The fluorophore of claim 6, wherein the linker comprises a DSPE (1,2-distearoyl-sn-glycero-3-phosphoethanolamine) group.

8. The fluorophore of claim 5, wherein the chain of PEG molecules has a molecular weight of greater than 1000 Da.

9. The fluorophore of claim 8, wherein the chain of PEG molecules has a stretched length of greater than 5 nanometers.

10. The fluorophore of claim 1 or claim 7, wherein the linker has a molecular weight greater than 3400 Da; and/or wherein the linker has a molecular weight less than 10000 Da.

11. The fluorophore of claim 1 or claim 8, wherein the length of the nanotube is between 50 and 1000 nm.

12. The fluorophore of claim 1 or claim 8, comprising at least 100 fluorescent entities, preferably at least 1000 fluorescent entities.

13. The fluorophore of claim 1 or claim 8, wherein the at least one fluorescent entity comprises a first fluorescent entity and a second fluorescent entity different from the first fluorescent entity.

14. The fluorophore of claim 1 or claim 8, further comprising a moiety selected from a group of a crown ether, a carbohydrate, a nucleic acid, a peptide, an oligonucleotide, a protein and an antibody, the linker being a first linker and a second linker linking the moiety to the nanotube.

15. The fluorophore of claim 1 or claim 8, wherein the at least one linker is covalently bonded to the at least one fluorescent entity via a reactive functional group.

## Patentansprüche

1. Ein Fluorophor, der Folgendes umfasst:
einen Träger, der ein Bornitridnanoröhrchen ist,
mindestens eine fluoreszierende Einheit und
ein amphiphiles Verknüpfungselement, das jede der mindestens einen fluoreszierenden Einheiten mit dem Träger verbindet, wobei das Verknüpfungselement ein Molekulargewicht von mehr als 1000 Da und/oder eine gestreckte Verknüpfungselementlänge von mehr als 5 Nanometern hat, wobei das Verknüpfungselement mindestens einen hydrophilen Anteil und einen hydrophoben Anteil umfasst und wobei der hydrophobe Anteil nicht-kovalent an den Träger gebunden ist und der hydrophile Anteil kovalent an die fluoreszierende Einheit gebunden ist.

2. Der Fluorophor gemäß Anspruch 1, wobei der hydrophile Anteil eine Kette wasserlöslicher Polymermoleküle einschließt, gebunden an den hydrophoben Anteil an einem ersten Ende, und die mindestens eine fluoreszierende Einheit an einem zweiten Ende an die Kette gebunden ist; wobei das Verknüpfungselement stärker bevorzugt eine DSPE- (1,2-Distearoyl-sn-glycero-3-phosphoethanolamin-)Gruppe und eine Kette von Polyethylenglykol-(PEG-)Molekülen umfasst.

3. Der Fluorophor gemäß Anspruch 1, wobei die mindestens eine fluoreszierende Einheit mindestens eines von einem Cumarin, einem Benzoxadiazol, einem Acridon, einem Acridin, einem Bisbenzimid, Indol, Benzoisochinolin, Naphthalen, Anthracen, Xanthen, Pyren, Porphyrin, Fluoreszein, Rhodamin, Bor-Dipyrromethen (BODIPY) und einem Cyaninderivat umfasst.

4. Ein Fluorophor gemäß Anspruch 1, der Folgendes umfasst:
ein Bornitridnanoröhrchen;
mindestens ein amphiphiles Verknüpfungselement, das einen hydrophoben Anteil und einen hydrophilen Anteil hat, wobei der hydrophobe Anteil nicht-kovalent an das Nanoröhrchen gebunden ist, wobei das mindestens eine amphiphile Verknüpfungselement ein Molekulargewicht von mehr als 1000 Da hat, und
mindestens eine fluoreszierende Einheit, kovalent an den hydrophilen Anteil des mindestens einen amphiphilen Verknüpfungselements gebunden.

5. Der Fluorophor gemäß Anspruch 4, wobei der hydrophile Anteil eine Kette wasserlöslicher Polymermoleküle einschließt.

6. Der Fluorophor gemäß Anspruch 5, wobei die wasserlöslichen Polymermoleküle Polyethylenglykol- (PEG-)Moleküle sind.

7. Der Fluorophor gemäß Anspruch 6, wobei das Verknüpfungselement eine DSPE- (1,2-Distearoyl-sn-glycero-3-phosphoethanolamin-)Gruppe umfasst.

8. Der Fluorophor gemäß Anspruch 5, wobei die Kette von PEG-Molekülen ein Molekulargewicht von mehr als 1000 Da hat.

9. Der Fluorophor gemäß Anspruch 8, wobei die Kette von PEG-Molekülen eine gestreckte Länge von mehr als 5 Nanometern hat.

10. Der Fluorophor gemäß Anspruch 1 oder Anspruch 7, wobei das Verknüpfungselement ein Molekulargewicht von mehr als 3400 Da hat und/oder wobei das Verknüpfungselement ein Molekulargewicht von weniger als 10000 Da hat.

11. Der Fluorophor gemäß Anspruch 1 oder Anspruch 8, wobei die Länge des Nanoröhrchens zwischen 50 und 1000 nm beträgt.

12. Der Fluorophor gemäß Anspruch 1 oder Anspruch 8, der mindestens 100 fluoreszierende Einheiten, vorzugsweise mindestens 1000 fluoreszierende Einheiten, umfasst.

13. Der Fluorophor gemäß Anspruch 1 oder Anspruch 8, wobei die mindestens eine fluoreszierende Einheit eine erste fluoreszierende Einheit und eine zweite fluoreszierende Einheit umfasst, die sich von der ersten fluoreszierenden Einheit unterscheidet.

14. Der Fluorophor gemäß Anspruch 1 oder Anspruch 8, der weiter einen Anteil umfasst, welcher gewählt ist aus einer Gruppe aus einem Kronenether, einem Kohlenhydrat, einer Nukleinsäure, einem Peptid, einem Oligonukleotid, einem Protein und einem Antikörper, wobei das Verknüpfungselement ein erstes Verknüpfungselement ist und ein zweites Verknüpfungselement den Anteil mit dem Nanoröhrchen verknüpft.

15. Der Fluorophor gemäß Anspruch 1 oder Anspruch 8, wobei das mindestens eine Verknüpfungselement über eine reaktionsfähige Funktionsgruppe kovalent an die mindestens eine fluoreszierende Einheit gebunden ist.

## Revendications

1. Fluorophore comprenant :
- un support qui est un nanotube de nitrure de bore ;
- au moins une entité fluorescente ;
- et un raccord amphiphile qui raccorde au support chacune des entités fluorescentes, au nombre d'au moins une, lequel raccord présente une masse molaire supérieure à 1000 Da et/ou une longueur supérieure à 5 nanomètres à l'état étiré, et lequel raccord comporte une partie hydrophile et une partie hydrophobe, étant entendu que cette partie hydrophobe est liée au support en mode non-covalent et que la partie hydrophile est liée en mode covalent à l'entité ou aux entités fluorescente(s).

2. Fluorophore conforme à la revendication 1, dans lequel la partie hydrophile comporte une chaîne de molécules polymères hydrosolubles attachée à la partie hydrophobe en une première extrémité, et l'entité fluorescente au nombre d'au moins une est attachée à cette chaîne en une deuxième extrémité, étant entendu que le raccord comprend de préférence un groupe 1,2-distéaroyl-sn-glycéro-3-phospho-éthanolamine (DSPE) et une chaîne de molécules de polyéthylène-glycol (PEG).

3. Fluorophore conforme à la revendication 1, dans lequel l'entité fluorescente au nombre d'au moins une comprend au moins l'une des suivantes : une coumarine, un benzoxadiazole, une acridone, une acridine, un bis-benzimide, indole, benzoisoquinoline, naphtalène, anthracène, xanthène, pyrène, porphyrine, fluorescéine, rhodamine, bore-dipyrrométhène (BODIPY), et un dérivé de cyanine.

4. Fluorophore conforme à la revendication 1, comportant :
- un nanotube de nitrure de bore ;
- au moins un raccord amphiphile comportant une partie hydrophobe et une partie hydrophile, dans lequel la partie hydrophobe est liée au nanotube en mode non-covalent, et lequel raccord amphiphile au nombre d'au moins un présente une masse molaire supérieure à 1000 Da ;
- et au moins une entité fluorescente liée en mode covalent à la partie hydrophile du raccord amphiphile au nombre d'au moins un.

5. Fluorophore conforme à la revendication 4, dans lequel la partie hydrophile comporte une chaîne de molécules polymères hydrosolubles.

6. Fluorophore conforme à la revendication 5, dans lequel les molécules polymères hydrosolubles sont des molécules de polyéthylène-glycol (PEG).

7. Fluorophore conforme à la revendication 6, dans lequel le raccord comprend un groupe 1,2-distéaroyl-sn-glycéro-3-phospho-éthanolamine (DSPE)

8. Fluorophore conforme à la revendication 5, dans lequel la chaîne de molécules de PEG présente une masse molaire supérieure à 1000 Da.

9. Fluorophore conforme à la revendication 8, dans lequel la chaîne de molécules de PEG présente, à l'état étiré, une longueur supérieure à 5 nanomètres.

10. Fluorophore conforme à la revendication 1 ou à la revendication 7, dans lequel le raccord présente une masse molaire supérieure à 3400 Da, et/ou le raccord présente une masse molaire inférieure à 10000 Da.

11. Fluorophore conforme à la revendication 1 ou à la revendication 8, dans lequel la longueur du nanotube vaut entre 50 et 1000 nm.

12. Fluorophore conforme à la revendication 1 ou à la revendication 8, qui comporte au moins 100 entités fluorescentes, et de préférence, au moins 1000 entités fluorescentes.

13. Fluorophore conforme à la revendication 1 ou à la revendication 8, dans lequel l'entité fluorescente au nombre d'au moins une comprend une première entité fluorescente et une deuxième entité fluorescente différente de la première entité fluorescente.

14. Fluorophore conforme à la revendication 1 ou à la revendication 8, comprenant en outre un fragment choisi dans l'ensemble constitué par un éther-couronne, un glucide, un acide nucléique, un peptide, un oligonucléotide, une protéine et un anticorps, étant entendu que le raccord est un premier raccord et qu'un deuxième raccord raccorde ce fragment au nanotube.

15. Fluorophore conforme à la revendication 1 ou à la revendication 8, dans lequel le raccord au nombre d'au moins un est lié en mode covalent à l'entité fluorescente au nombre d'au moins une par l'intermédiaire d'un groupe fonctionnel réactif.
